# EUROPEAN PATENT SPECIFICATION

(11) **EP 0 435 911 B1**
(45) Date of publication and mention of the grant of the patent: **13.03.1996**
(21) Application number: 89910564.7
(22) Date of filing: 13.09.1989
(51) Int. Cl.: C12N 5/00

(54) **CELL CULTURE MEDIUM FOR ENHANCED CELL GROWTH, CULTURE LONGEVITY AND PRODUCT EXPRESSION**
ZELLENZUCHTMEDIUM FÜR ERHÖHTES ZELLENWACHSTUM, ZUR ERHÖHUNG DER LANGLEBIGKEIT UND EXPRESSION DER PRODUKTE
MILIEU DE CULTURE DE CELLULES POUR L'AMELIORATION DE LA CROISSANCE DES CELLULES, DE LA LONGIVITE DE LA CULTURE ET DE L'EXPRESSION DU PRODUIT

(30) Priority: 23.09.1988 US 248634
(43) Date of publication of application: 10.07.1991
(73) Proprietor: CETUS ONCOLOGY CORPORATION, Emeryville California 94608 (US)
(72) Inventor: HOWARTH, William, Castro Valley, CA 94552 (US); MAIORELLA, Brian, Oakland, CA 94618 (US); INLOW, Duane, Oakland, CA 94610 (US)
(74) Representative: Bizley, Richard Edward
(86) International application number: US8903986
(87) International publication number: WO9003430

(56) References cited:
- EP-A- 0 249 557
- WO-A-87/00195
- GB-A- 2 196 348
- US-A- 4 560 655
- US-A- 4 657 866
- Biotechnology Letters, Vol. 9, No. 10, September 1987; Y.T. Luan et al.: "Strategies to extend longevity of hybridomas in culture and promote yield of monoclonal antibodies ", pp. 691, 693, 696
- Immunology Letters, Vol. 7, 1984; J. Kovar et al.: "Serum-free medium for hybridoma and parental myeloma cell cultivation: a novel composition of growth-supporting substances. ", pp. 339-345
- Journal of Immunological Methods, Vol. 97, 1987; S.P.C. Cole et al.: "Growth of human X human hybridomas in protein-free medium supplemented with ethanolamine", pp. 29-35
- Journal of Immunological Methods, Vol. 94, 1986; John P. Tharakan et al.: "Hybridoma growth and antibody secretion in serum-supplemented and low protein serum-free media ", pp. 225-235
- Proc. Natl. Acad. Sci. USA, Vol. 79, February 1982; Hiroki Murakami et al.: "Growth of hybridoma cells in serum-free medium: Ethanolamineis an essential component", pp. 1158-1162

## Description

This invention is in the general field of animal cell culture. More particularly, the invention concerns improved media for the cultivation of animal cells and the production of natural and recombinant products derived therefrom.

To date, efforts have been undertaken to develop culture conditions to maximize cell culture growth and thereby increase resultant product yield. Early work in the development of animal cell culture media focused on the formulation of such media to achieve rapid cell proliferation (White, P.R., 1946, Growth, 10:231-289, and Waymouth, C., 1974, J. Natl. Cancer Inst., 53:1443-1448). Such media incorporate specific nutrients especially sugars, amino acids, vitamins, salts, and in some cases trace metal ions, purines, and pyrimidines. These media are most often supplemented with animal serum. Today some of the more widely used basal media for mammalian cell cultures include Hams F-12, Dulbecco's modified Eagle's medium (DME), RPMI 1640, and Iscove's modified DME.

Production of human monoclonal antibodies using hybridoma cell lines will be used in this application as an example for product expression in cell culture.

Culture media have been previously described which were developed specifically for low serum and serum-free mammalian cell cultures for production of monoclonal antibodies. One such serum-free medium is disclosed in European Patent Publication 076,647, published April 13, 1983. Other media have been developed by changing levels of supplements such as trace elements, and vitamins and incorporating purified protein hormone additives. References to such media include, for example, Barnes, D., et al., 1980 Cell, 22:649-655; Cleveland, W.L., et al. 1983, J. Immunol. Meth., 56:221-234; Iscove, N., et al., 1978, J. Exp. Med., 147:923-933; Kawamoto, T., et al., 1983, Analytical Biochemistry, 130:445-453; Kovar, J., et al., 1984, Immunology Letters, 7:339-345; Murakami, H., et al., 1983, Argic. Biol. Chem., 47(8):1835-1840; Murakami, H., et al., 1982, Proc. Natl. Acad. Sci. USA, 79:1158-1162; Muzik, H., et al., 1982, In Vitro, 18:515-524; and Wolpe, S.D., "In Vitro Immunization and Growth of Hybridomas in Serum-Free Medium", in J.P. Mather, ed., Mammalian Cell Culture, Plenum Press, New York, 1984; Hagiwara, H., et al., 1985, 117-122 in H. Murakami et al. (eds) Growth and Differentiation of Cells in Defined Environment, Springer-Verlag, Berlin, 1985; Tharakan, J.P., et al., 1986, J. Immunol. Meth., 94:225-235; Cole, S.P.C., 1987, J. Immunol. Meth., 97:29-35; McHugh, Y.E., 1983, BioTechniques, June/July issue:72-77. Components which are common to most if not all these media include glucose at concentrations up to 4.5 g/L, glutamine at concentrations of 2-4 mM, choline generally at about 1-4 mg/L, tryptophan and other amino acids. Tryptophan is generally present at concentrations less than 20 mg/L. Several of these media also contain the growth factors insulin and transferrin.

Efforts to increase antibody yield have focused primarily on means to optimize cell growth and cell density. As a general point of reference, antibody titres from murine hybridoma cell lines are highly variable from cell line to cell line and range typically from 10 to 350 mg/L (Lambert, K.J., et al., 1987, Dev.Indust. Microbiol, 27:101-196). Human monoclonal antibody expression from human/human or human/mouse fusions are also highly variable from cell line to cell line and range typically from 0.1 to 25 mg/L (Hubbard, R., Topics in Enzyme and Fermentation Biotechnology, Chapt. 7:196-263, Wisemand, A., ed., John Wiley & Sons, New York (1983). These values are indicative of culture conditions that are optimized for cell growth.

Another approach from the literature to increasing product production is to achieve high cell densities by cell recycle or entrapment methods. Examples of these methods include hollow fiber reactors (Altshuler, G.L., et al., 1986, Biotech. Bioeng., XXVIII:646-658; ceramic matrix reactors (Marcipar, A., et al., 1983, Annals. N.Y. Acad.Sci., 413:416-420; Nature, 302:629-630); perfusion reactors (Feder, J., et al., 1985, American Biotech. Laboratory, III:24-36) and others.

While a variety of methods to increase product expression from cell culture are being explored, the primary focus is still on the optimization of cell growth. In typical culture media the culture dies rapidly after maximum cell density is reached.

Another example from the literature documents that, at least for some cell lines, product (monoclonal antibody) production proceeds even after a culture stops growing (Velez, D., et al., 1986, J. Immunol. Meth., 86:45-52; Reuveny, S., et al., 1986, ibid at 53-59). Arathoon, W., et al., 1986, Science, 232:1390-1395 reported that a 1,000 liter hybridoma fermentation produced about 80 grams of monoclonal antibody during the growth phase and another 170 grams of antibody during an extended stationary/death phase. J. Birch, et al., (European Patent Application No. 87/00195, 1987) describe a procedure of Fed-batch culture wherein nutrients are added to a culture over time and culture longevity is increased. Final antibody titres from the culture are thus increased.

Thus, it will be appreciated that there is a critical need for media that will support the growth of animal cells and stimulate the production of products, including antibodies, and other natural or recombinant protein products to greater levels than can be realized using media that are currently available.

Accordingly, the invention presented herein describes a protein-free supplement (the "Primary Supplement"), which when added to standard cell culture media, enhances cell growth, culture longevity and product expression. Examples are given showing that this supplement is particularly effective in production of antibodies using hybridoma cell lines in serum-free culture, where final antibody titre is increased in one example from 80 mg/L to over 250 mg/L. The Primary Supplement is a hitherto unrecognized synergistic combination of standard medium components which are beneficial when added in the prescribed combination, and consist of 1) glutamine, 2) phospholipid precursors, including minimally both choline and ethanolamine, 3) tryptophan, and 4) additional amino acids as required for a particular cell line and product to be produced. When added individually to standard media, these components have little effect, but are extremely effective when added together in the prescribed combination, and the deletion of any of the prescribed components diminishes the desired effect. Several of the components in the Primary Supplement are added to concentrations hitherto considered to be unnecessary or inhibitory. When added together in the prescribed combination, the supplement enhances cell growth, increases culture longevity by maintaining cells in a pseudo-stationary phase wherein product expression continues, and thus results in a significant increase in final product titre.

A second aspect of the invention is a description of several additional components, (the "Class I reagents"), which when added individually, or in various combinations to the Primary Supplement result in a further improvement to culture growth and/or product expression. Included in this class of additional components are reducing agents, trace metal ions, and/or vitamins.

A third aspect of the invention is the formulation of serum-free and serum supplemented media containing the supplement, as well as in media supplemented with lipids and can be used with the addition of agents to induce solute stress.

A further aspect of the invention is a method of growing cells employing the media supplements described herein such that they can be included in medium at the start of culture, or can be added in a fed-batch or in a continuous manner. The resulting media can be used in various cultivation methods including, but not limited to, batch, fed-batch, hemostat and perfusion, and with various cell culture hardware including, but not limited to, stationary flasks, agitated flasks, spinner flasks, stirred fermentors, airlift fermentors, membrane reactors (including hollow fiber, flat membrane plate, external loop perfusion), reactors with cells retained on a solid support of immobilized/entrapped as in microporous beads, and any other configuration appropriate for the growth or maintenance of the desired cell line.

Figure 1 shows the growth properties and levels of antibody produced by D234 in a typical commercially available media, Ventrex HL-1.

Figure 2 shows the growth properties and levels of antibody produced by D234 in a standard serum free medium composition representative of compositions described in the cell culture literature.

Figure 3 shows the growth properties and levels of antibody produced by D234 in media supplemented with reagents of the Primary Supplement.

Figure 4 shows the growth properties and levels of antibody produced by D234 in media supplemented with reagents of the Primary Supplement and a Class I reagent.

Figure 5 shows the growth properties and levels of antibody produced by D234 in media supplemented with reagents of the Primary and Class I Supplement and used in combination with lipid supplementation.

Figure 6 shows the hybridoma D234 grown in Fed-batch with media supplemented with reagents of the Primary and Class I Supplements and lipids.

The following reference is referred to throughout this application, and is presented here for the convenience of the reader. Ian Freshney (Culture of Animal Cells -- A Manual of Basic Technique, Alan R. Liss, NY, 1987) tabulates compositions of typical basal media for use with serum (Table 7.5:74-75) and typical serum-free media compositions (Table 7.6:76-78) described in the literature for culture of a wide range of animal cells and expression of products therefrom. These tables are incorporated by reference.

The invention described herein is first concerned with a protein-free supplement compatible with standard cell culture media to enhance cell growth, culture longevity and product expression. The Primary Supplement is favored, and has advantages that arise, at least in part, by a hitherto unrecognized synergistic interaction of the reagents employed Iscove (N.N. Iscove, Culture of Lymphocytes and Hemopoietic Cells in Serum-Free Medium:169-185 in Methods for Serum-Free Culture of Neuronal and Lymphoid Cells) describes the standard method for optimizing cell culture media wherein "the effect of individually doubling and quadrupling the concentration of each component was examined ...". This approach has been widely used in the cell culture field, which fails to recognize the possibility that components may act synergistically and that it may be necessary to combine supplements of several components to see a desired effect.

Components selected from a second group of cell culture reagents (the Class I reagents) when combined with the Primary Supplement can synergize to produce a more favorable cell culture supplement having especially beneficial effects on cells grown to high densities.

Before a detailed description of the classes of reagents is presented and the media supplements that can be formulated therefrom, a brief definition of some of the technical terms used throughout this application will facilitate understanding the nature of the invention.

### Definitions

"Basal medium" is defined herein to include a nutrient mixture of inorganic salts, sugars, amino acids, and, optionally, vitamins, organic acids and/or buffers or other well known cell culture nutrients. These reagents are necessary to suppon cell growth and reproduction. The preferred basal medium which is a component of the instant cell growth media compositions contains neither serum, nor proteins. A wide variety of commercially available basal media are well known to those skilled in the art, and include Dulbeccos' Modified Eagles Medium (DMEM), Roswell Park Memorial Institute Medium (RPMI), Iscove modified Dulbeccos' medium and Hams medium.

The term "hybridoma", refers to a hybrid cell line produced by the fusion of an immortal cell line of immunologic origin, and an antibody producing cell. The term is meant to include progeny of heterohybrid myeloma fusions, which are the result of a fusion with human cells and a murine myeloma cell line subsequently fused with a plasma cell, referred to in the art as a trioma cell line. Additionally, the term is meant to encompass virtually any immortalized hybrid cell line which produced antibody such as, for example, quadromas. Milstein, C., et al., 1983, Nature, 537:3053. Moreover, the hybrid cell lines can be of virtually any species, including human and mouse.

The term "microemulsion" refers to a lipid mixture emulsified substantially without the aid of proteinaceous materials such as those found in serum, particularly serum albumin. Generally, emulsifiers described in PCT Application No. WO 89/01027, published January 9, 1989, will be employed to form the microemulsion. This patent application is hereby Incorporated by reference in its entirety. Generally, pluronic polyols are favored as the emulsifier.

As used herein the term "solute stress" refers to the addition of solutes in such concentrations that produce a growth inhibitory effect or reduced final cell density, that is, a growth rate or maximum cell density less than that determined for optimal growth. However, the level of product expressed at this reduced growth level is comparatively greater than that level of expression achieved at the optimal growth rate owing to an increase in specific (per cell) product expression rate or an increase in longevity of the culture. Generally, solutes and methods described PCT Application No. WO 89/04867, published June 1, 1989, will be employed. This application is hereby incorporated by reference in its entirery.

"Pseudo-Stationary phase" refers to a period of culture growth occurring after the exponential growth phase and wherein the rates of cell growth and death are similar such that the viable cell concentration changes only relatively slowly with time (as compared to during the exponential growth or the death phase).

"Cell growth hormones" or "growth factors" is meant to encompass a large number of molecules either naturally occurring or genetically engineered, as well as fragments, derivatives or modifications thereof, that stimulate the growth, or support the maintenance of cells in defined media Examples of the former include transferrin and insulin, among others. Examples of growth factors include nerve growth factor, epidermal growth factor, fibroblast growth factor, and endothelial cell growth factor, among others. It is important to note that different animal cell lines may vary in their requirements for one or more of these molecules, and that the optimal hormones or growth factors, or a mixture of the same, are readily determined using standard cell culture techniques.

The instant invention presents cell culture media supplements that are compatible with the general growth and maintenance requirements of animal (especially mammalian) cells in vitro, and particularly enhance or support the expression of differentiated properties associated with specific cell types, for example, an elevated production of monoclonal antibodies by hybridoma cell lines. Thus, it will be appreciated that the instant supplements have widespread applications, being generally useful for routine culture of cells, as well as being employed in those instances when there are desired great amounts of a natural or recombinant cellular product that is produced by cells in culture.

### Primary Supplement

The Primary Supplement of the current invention consists of a class of reagents that includes, in combination, 1) glutamine, 2) phospholipid precursors including preferablv choline and ethanolamine, 3) tryptophan, and 4) additional amino acids as required for the particular cells lines.

### a) Glutamine:

In the Primary Supplement, glutamine is included at over 5 mM, and preferably at 8-20 mM. It is important to note that these concentrations are significantly above those typically found in cell culture media.

Glutamine is recognized as both an amino acid building block for protein synthesis and as a energy source in cell culture (W.L. McKeehan, Glycolysis, Glutaminolysis and Cell Proliferation, Cell Biology Intro. Reports, 1982, 6(7):635-650, LJ. Reitzer, et al., Evidence that Glutamine, Not Sugar, Is the Major Energy Source for Cultured HeLa Cells, J. Biological Chemistry, 1979, 254(B):2669-2676, H.R Zielke, et al., Reciprocal Regulation of Glucose and Glutamine Utilization by Cultured Human Diploid Fibroblasts, J. Cell Physiol., 1978, 95:41-48.

Reference to the summary of typical media compositions tabulated in Freshney, above, shows that glutamine is typically included at 2 or 4 mM in standard serum-supplemented and serum-free medium formulations. In contrast as mentioned above, the composition of the instant invention contains elevated amounts of glutamine above 5 mM, and preferably about 8-20 mM.

Both glutamine metabolism as well as spontaneous decomposition of glutamine (G.L Trisch, et al., Spontaneous Decomposition of Glutamine in Cell Culture Media, Experimental Cell Research, 1962, 28:360-364) result in the release of ammonium ion which is widely described in the literature as toxic to either cell growth or protein production (S. Reuveny, et aL, Factors Affecting Cell Growth and Monoclonal Antibody Production in Stirred Reactors, J. Immunl. Methods, 1986, 86:53-59) and some researchers have argued that glutamine when added at high concentrations will have a toxic effect (indirectly through increased production of ammonium ion). Some researchers have advocated minimizing the concentration of glutamine present in the culture by adapting the culture to grow in the absence of glutamine and with glutamic acid as an alternate substrate (J.B. Griffiths, et al., The Uptake of Amino Acids by Mouse Cells (Strn LS) During Growth in Batch Culture and Hemostat Culture, The Influence of Cell Growth Rate, Proc. Roy Soc. London, 1967, 168:421-438), or by utilizing slow addition of glutamine throughout the time course of the culture to maintain a relatively constant low concentration of glutamine (M.W. Glacken, et al., Reduction of Waste Product Excretion via Nutrient Control: Possible Strategies for Maximizing Product and Cell Yields on Serum in Cultures of Mammalian Cells, Biotechnol. Bioeng., 1986, 28:1376-1389).

Based on the forgoing discussion, it will be realized that for cell lines which have been adapted to grow with glutamate or other substitute for glutamine that the methods of the current invention are still applicable, except that elevated levels of glutamate or other substitute would be included in the supplement in place of glutamine. For cell lines that have been adapted to grow with low concentrations of glutamine maintained by glutamine addition throughout the culture, the methods of the current invention are also applicable except that the increased quantity of glutamine will be added gradually over the course of the culture.

Recently, some researchers have found that addition of supplemental glutamine late in culture can increase culture longevity for some cell lines (S. Reuveny, et al., Factors Affecting Cell Growth and Monoclonal Antibody Production in Stirred Reactors, J. Immunol. Methods, 1986, 86:53-590).

We have found that supplemental glutamine can be added either at the start of culture, or during the course of culture with the total of glutamine added to about 5-40 mmoles and preferably about 8-20 mmoles per liter. We believe that late in culture, in a post exponential growth pseudo-stationary phase, glutamine can be a major energy source for the cells, (i.e. glutamine consumption continues while glucose consumption may decline). We find that glutamine supplementation is necessary, but not sufficient, to achieve the desired increase in culture longevity. To achieve the optimal performance, glutamine must be added in combination with the other reagents of the Primary Supplement.

### b) Phospholipid Precursors:

The Primary Supplement also contains phospholipid precursors, selected from the group including but not limited to, serine, inositol, choline, ethanolamine, and glycerol. While these components are all phospholipid precursors, they also have other biochemical roles, and this invention should not be considered as being limited by any proposed hypothesis of a mechanism of action.

Serine is considered an essential amino acid, and inositol an essential vitamin. Most cell culture media contain serine and inositol at adequate levels for their roles as phospholipid precursors and other biochemical roles.

Choline is Included as a vitamin in most media at 1-4 mg/L. However, on occasion, choline has been used at higher concentrations. For instance, to grow cells of nonlymphoid origin, Ham's (R.G. Ham, Clonal Growth of Mammalian Cells in a Chemically Defined Synthetic Medium, Proc. natl. Acad. Sci. USA, 1965, 53:288-293) includes choline at 15 mg/L in a serum-free medium for clonal growth of Chinese Hamster Ovary Cells. J.Birch, et al. (J. Cell Sci., 1969, 5:135-142) includes choline at 20 mg/L in a serum containing medium for growth of mouse fibroblasts. Waymouth's (C. Waymouth, Rapid Proliferation of Sublines of NCTC Clone 929 Mouse Cells in a Simple Chemically Defined Medium, J. Natl. Cancer Inst., 1959, 22:1003-1017) medium MB 752/1 for culture of mouse L929 fibroblast connective tissue cell line is exceptional in including choline at 250 mg/L. Although Ham, Birch and Waymouth developed media to suppon cell growth, they did not study the production of biological products produced by cells grown in the media. We have found that cells, preferably antibody secrting cells, grow and secrete maximal amounts of antibody in media containing choline supplemented to a level of greater than about 4 mg/L, and preferably at approximately 4-40 mg/L in combination with the other reagents of the Primary Supplement. At these concentrations, choline does not become limiting and is without apparent toxicity.

Ethanolamine is not typically included in serum supplemented media. Murakami's (H. Murakami, et al., Proc. Natl. Acad. Sci. USA, 1982, 79:1158-1162) demonstrates that ethanolamine at 20 uM is stimulatory to growth of a murine hybridoma cell line. Tharakan, et al., J. Immunol. Methods, 1986, 94:225-235; Cole, et al., J. Immunol. Methods, 1987, 97:29-35; and Kovar, et al., Immunol. Letters, 1984, 7:339-345 also found ethanolamine to be stimulatory to growth of several murine hybridoma cell lines. None of these researchers combined elevated levels of ethanolamine with elevated levels of glutamine, choline, tryptophan and other amino acids as we have found necessary for optimum product expression. Several other serum-free media do not include ethanolamine (T. Chang, et al., J. Immunol. Methods, 1980, 39:369-375) and a review by Iscove (N.N. Iscove, Culture of Lymphocytes and Hemopoietic Cells in Serum-Free Medium p. 169-185 in Methods for Serum-Free Culture of neuronal and Lymphoid Cell Lines, Alan R. Liss Inc., NY 1984) identifies choline and inositol as essential, but does not mention ethanolamine. We have found that ethanolamine is effective when supplemented to a level of approximately 1-10 mg/L, and can be included at up to at least 10 mg/L without apparent toxicity.

It should be understood that choline and ethanolamine can be provided in various forms including phosphocholine and phosphoethanolamine or phosphatidylcholine and phosphatidylethanolamine. The relative effectiveness of these various forms will depend on the ability of the specific cell line to take up (transport) and metabolize the complex forms.

Supplementation of standard media with phospholipid precursors alone is not sufficient to achieve the desired maximum extension of culture longevity and product production. Rather, phospholipid precursors at in synergy with the other components described.

### c) Tryptophan:

Tryptophan is recognized as an essential amino acid and is included in typical serum-supplemented and serum-free media at 2-20 mg/L, with the exception of Waymouth's MB752/1 medium for L-929 cells where it is present at 40 mg/L. Several researchers have attempted to optimize media by supplementation with increased levels of each amino acid added separately. Typical of these, Barns and Iscove (N.N. Iscove, Culture of Lymphocytes and Hemopoietic Cells in Serum-Free Medium p. 169-185 in Methods for Serum-Free Culture of Neuronal and Lymphoid Cells) have both tested supplementation of media with each of the standard amino acids, and neither found addition of tryptophan at greater than standard levels to be stimulatory. We have found that tryptophan supplementation is essential to achieving the desired increase in culture longevity and product titre. For optimal effect, tryptophan is supplemented to levels higher than those typically taught in the literature, or greater than 20 mg/L, and can be added at up to at least 100 mg/L without toxic effect.

Supplementation of standard media with tryptophan alone is not sufficient to achieve the desired effect. Rather, tryptophan at elevated concentrations acts in synergy with the other components of the Primary Supplement to achieve the desired maximum extension of culture growth and longevity, and product production.

### d) Other Amino Acids:

The recommended Primary Supplement also includes a formulation of amino acids determined to be desirable for expression of a product form a particular cell line.

Amino acids are the essential building blocks for protein synthesis. Supplementation of medium with the components specified above provides the basis for increased culture longevity, and hence, in a significantly increased period for product production. Under these conditions, amino acids required for cell maintenance and product synthesis can become depleted. To maximize final product titre, the levels of these amino acids must be increased so as to not become limiting. Amino acid analysis of spent medium using techniques which are known to the analytical chemist (D.H. Speckman, et al., Automatic Recording Apparatus for Use in the Chromatography of Amino Acids, Analytical chemistry, 1958, 30:1190-1206) can be used as a tool to identify those amino acids which are depleted during culture and require supplementation.

Iscove (N.N. Iscove, Culture of Lymphocytes and hemopoietic Cells in Serum-Free Medium p. 169-185 in Methods for Serum-Free Culture of Neuronal and Lymphoid Cells) and others have tested supplementation of media with each of the standard amino acids. Typical of the literature, Iscove found that addition at greater than standard levels of all amino acids tested, (except cystine), was not stimulatory.

Luan (Y.T. Luan, et aL, Strategies to Extcnd Longevity of Hybridomas in Culture and Promote Yield of Monoclonal Antibodies) describes a fed batch strategy, consisting of adding a supplement containing glutamine, essential amino acids (including tryptophan), vitamins (including choline) and serum at various time points during the culture of a murine hybridoma cell line. The medium to which additions was made consisted of DMEM supplemented with fetal calf serum This method resulted in an increase in culture longevity and increase in final antibody titre. Similarly, Birch (J.R. Birch, et al., Animal Cell Culture, EPA PCT/GB86300383, 1986) describes fed-batch culture adding a supplement containing glucose, glutamine essential and non-essential amino acids (including tryptophan) during the culture of another murine hybridoma cell line. The medium to which additions was made was also DMEM supplemented with fetal calf serum.

It is important to note regarding Luan and Birch that neither included in their media ethanolamine which we have found to be a highly favored phospholipid precursor for the optimum cell growth and product production effect. Further, while we have found that our supplement can be used effectively in fed-batch and continuous culture, we have found (unlike Luan and Birch) that inclusion of the supplement at the start of culture is also effective (i.e. in contrast to Luan and Birch, nutrient addition during culture is not obligatory using the supplement of the current invention). Additionally, applicants' supplement can be used advantageously with serum-free media. Also differentiating the current work, neither Luan and Birch included a defined reducing agent, such as MTG which we have identified, as discussed below, as a preferred embodiment which can result in a further increase in cell product expression.

### Class I Reagents

The reagents of the Primary Supplement can be combined with additional reagents to produce media having more favorable cell growth and product expression properties. These Class I reagents include, but are not limited to, reducing agents, trace metal ions and/or vitamins. Components of the Class I reagents can be added individually or in combination with the complete set of reagents of the Primary Supplement. The utility of these additional Class I reagents will vary depending on the cell line and basal medium composition.

### a) Reducing Agents:

The metabolism of glutathione and related sulfhydryl species has been reviewed by Meister (A. Meister, Selective Modification of Glutathione Metabolism, Science, 1983, 220:472-477). Yamane (I. Yamane, et al., Effects of Sulfhydryl Groups and Oxygen Tension on the Cell Proliferating Activity of Bovine Serum Albumin in Culture, Cell Structure and Function, 1982, 7:133-143) shows that addition of reducing agents can protect cultures from damage associated with high oxygen tension.

Some serum-supplemented media (such as RPMI 1640) contain added glutathione (GH) at 1-15 mg/L. Other reducing agents are not generally included in serum-supplemented media. For example, beta-mercaptoethanol (B-ME) and monothioglycerol (MTG) are not typically included in cell culture media, and are not Included In any of the media tabulated by Freshey above. In contrast, glutathione or dithiothreitol (DTT) is included in MCDB110 serum-free medium, but they are absent from several other serum-free formulations (CDB 170, MCDB 153, WAJC, KITES, etc.). Iscove's serum-free medium does not contain GH or DTT.

It is important to note that, although there are reports of media containing reducing agents, that none of these media contain all of the reagents of the Primary Supplement. For instance, Iscove (N.N. Iscove, Culture of Lymphocytes and Hemopoietic Cells in Serum-Free Medium, p 169-185, in Methods for Serum-Free Culture of Neuronal and Lymphoid Cells) recommends supplementation of his IMDM medium with either B-ME (5 x 10⁻⁵M) or MTG 7.5 x 10⁻⁵M. Iscove's medium does not, however, contain ethanolamine or the elevated levels of glutamine, tryptophan and other amino acids required for the synergistic effect observed for cell growth and product expression attributed to reagents of the Primary Supplement. Further, Kawamoto (T. Kawamoto, Anal, Biochem., 1983, 130:445-453) has supplemented a basal medium with several components including ethanolamine (10 µM) and B-ME (10 µM). Kawamoto's media do not, however, contain the elevated levels of glutamine, tryptophan and other amino acids required for the synergistic effect. Likewise, Kovar (J. Kovar, et al, Immunol. Letters, 1984, 7:339-345) developed a serum-free medium containing ethanolamine (20 µM), but this medium again contains low levels of glutamine, tryptophan and other amino acids and does not result in the synergy which we have discovered. Kovar (J. Kovar, et al., Serum-Free Medium for Hybridoma and Parental Myeloma Cell Cultivation: A Novel Composition of Growth Supporting Substances) tested β-mercaptoethanol as a possible component in their ethanolamine supplemented RPMI-1640 based serum-free medium. This medium did not contain elevated levels of glutamine, tryptophan, other amino acids, or choline, which we have found favors the optimum synergistic effect. In the absence of these components, Kovar and Frank found B-ME not to be useful and deleted it from their final formulation.

We have found that supplementation of culture media with a reducing agent/sulfhydryl compound can result in an increase in culture longevity and product titre. However, such supplementation is maximally effective when in combination with the complete Primary Supplement. Reducing agent/sulfhydryl compounds for use include β-mercaptoethanol, monothioglycerol (MTG), dithiothreitol, glutathione, thioglycolate, and cystine. More preferred are thiol molecules which have hydroxyl group(s) such as (β-mercaptoethanol) B-ME, (monothioglycerol) MTG and (dithiothreitol) DTT. Most preferred are mono-thiol compounds of this class such as B-ME and MTG. These are effective at concentrations above 0.1 mg/L and are not toxic at up to at least 10 mg/L. A preferred concentration is 0.5 - 5 mg/L.

### b) Metal Ions:

Metal ions are essential for animal cell culture and are included in typical media as components of salt and trace element mixtures, or as components of undefined supplements such as serum (K. Higuchi, "Cultivation of Animal Cells in Chemically Defined Media - A Review", Advan. Appl. Microbiol., 16:111-136). In media supplemented with the reagents of the Primary Supplement, high cell densities can be reached such that availability of certain metal ions, if included only at the levels incorporated in standard media designed to support lower cell densities, can become limiting. Therefore, in a preferred embodiment of the current invention, metal ions are also included in the supplement as found to be necessary for a particular cell line. At the high concentrations that can be required, solubility limits of some metal ions may limit the maximum concentration that can be added with beneficial effect. Therefore, a further preferred embodiment is to supply necessary metal ions along with a suitable chelating agent. The chelating agent must be nontoxic at the concentrations added, and must bind the required metal ions in a reversible manner such that they may be held in solution at adequate concentration, but will be delivered to the cells in an active form.

Analysis of the spent medium using techniques which are well known to the analytical chemist ("Flame Photometry", Chpt. 11, in H. Willard, et al., Instrumental Methods of Analysis, Van Nostrand Co., 1965) can be used as a tool to identify those metal ions which are utilized and may require supplementation. Some metal ions which may require supplementation include, but are not limited to, calcium, magnesium, molybdenum, cobalt, copper, manganese, zinc, selenium and iron. A more extensive list is given by Hamilton and Ham (W. Hamilton, et al., Clonal Growth of Chinese Hamster Cell Lines in Protein-Free Media, In Vitro, 1977, 13(9):537-547).

Various metal chelators have been used in cell culture. Natural proteins which can serve this function include transferrin and ferritin, especially to chelate iron, and albumin to chelate a variety of multivalent metal ions. Some other chelators which have been used especially to chelate iron include pyridoxyl isonicotinoyl hydrazone, choline citrate, citrate (C. III, T. Brehm, et al., Species Specificity of Iron Delivery in Hybridomas, In Vitro Cell. Develop. Biol., 1988, 24(5):413-419) and acetylacetonate (L. Rasmussen, et al., Utilization of Iron Complexes in an Animal Cell, J. Cellular Physiol., 1985, 122:155-158). We have found citrate at about 1-10 mM to be an effective chelator capable of supplying several multivalent metal ions. In addition, a variety of other organic acids such as malic acid, succinic acid, fumaric acid and alpha ketoglutaric acid are effective chelators.

### c) Vitamins:

Vitamins are essential for animal cells in culture and are generally included in cell culture media (K. Higuchi, Cultivation of Animal Cells in Chemically Defined Media - A Review p. 111-136 in Advan. Appl. Microbiol., 1973, 16:111). In media supplemented only with the Primary Supplement composition, high cell densities can be reached such that availability of certain vitamins, if included only at the levels incorporated in standard media designed to support lower cell densities, can become limiting. Therefore, in a further preferred embodiment of the current invention, vitamins may also be included in the supplement as found to be necessary for a particular cell line. Vitamins which may become limiting include, but are not limited to, p-amino-benzoic acid, biotin, folic acid, folinic acid, nicotinamide, pantothenate, pyridoxine, riboflavin, flavin adenine dinucleotide, ascorbic acid, thiamine and vitamin B-12.

Having generally described the media supplements of the instant invention, several limitations associated with their use, as well as ways to circumvent the limitations warrant discussion.

First, although it is convenient to define classes of reagents that can be readily combined with culture media that is fed to cells without later during the culture period having to refeed the cells, it will be appreciated by those skilled in the art that similar favorable effects can be gained by adding individual reagents to the culture media during the culture period, so as to maintain their concentrations at the levels provided by the supplements. This is most apparent with regard to glutamine, which can be added in combination with the other reagents of the Primary Supplement, or omitted and individually added and maintained at the desired level.

Secondly, the concentrations of reagents that are used in the various supplements will vary over the ranges stated, and are a function of both the cell culture media to which the supplements are added, as well as the particular cell type cultured. The optimal concentrations of the reagents are readily determined by those skilled in the art.

Thirdly, with the exception of reagents of the Primary Supplement, all of which must be present in the supplements for maximum cell culture benefit, only one of the Class I reagents need be present for advantageous results.

Fourthly, it will be realized that for optimal results, the basal medium to which the supplement is added must be appropriate for the cell line of interest, with key nutrients available at adequate levels to enhance cell growth or product expression. Thus, for example, it may be necessary to increase the level of glucose (or other energy source) in the basal medium, or to add glucose (or other energy source) during the course of culture, if this essential energy source is found to be depleted and to thus limit cell growth or product expression.

The following examples illustrate the invention, but are not intended to limit it in any manner. For instance, hybridomas and antibodies are illustrative of cell lines that can be successfully grown and harvested, respectively in media supplemented with the instant compositions. However, such media are not limited to growing hybridomas or harvesting antibody, but rather can be used to grow a side variety of cells that produce a broad range of products.

### Example 1

### A Representative Commercial Medium Formulation

Ventrex HL-1 is a medium, representative of state-of-the-art commercially available media for cell culture. Product literature indicates that this medium "was designed for use in the culture of hybridomas and lymphoid cells". The medium is claimed to be effective for growth of many transformed and established cell lines, and especially for production of monoclonal antibodies from murine and human hybridomas. A list of 55 cell lines grown successfully in this medium is presented in Current Concepts, 1(1):5, published by Ventrex, 217 Read St, Portland, Maine.

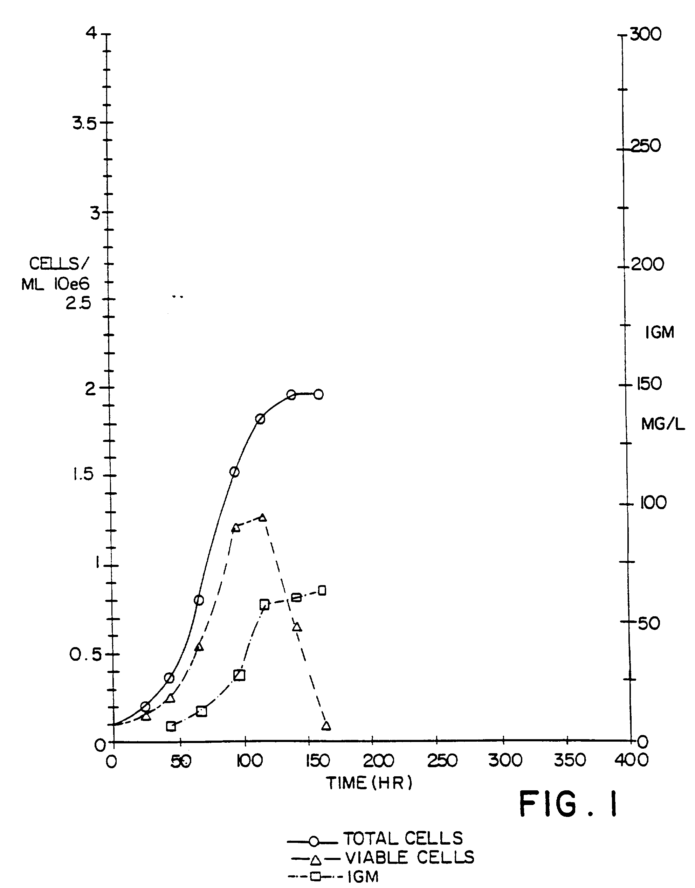

Figure 1 shows the growth and product expression from a hybridoma cell line, D-234, producing a human monoclonal antibody in HL-1 medium. The maximum viable cell density of 1.3 x 10⁶ cell/ml is reached at 120 hours and the culture dies rapidly thereafter. The maximum total cell density is 2 x 10⁶ cells/ml. The final antibody titre is 70 mg/L.

The hybridoma D-234 is on deposit with the American Type Culture Collection with Accession No. HB 8598.

The hybridoma produces an IgM antibody which was assayed using standard ELISA Techniques.

### Example 2

### Serum-Free Hybridoma Medium Formulation

Table I, column 1, shows a typical serum-free medium formulation (50% RPMI, 50% DME, bovine insulin (5 mg/L), human transferrin (5 mg/L), selenium (5 µg/L), 0.1% Pluronic polyol (F68). This medium is similar to several compositions reported in the literature.

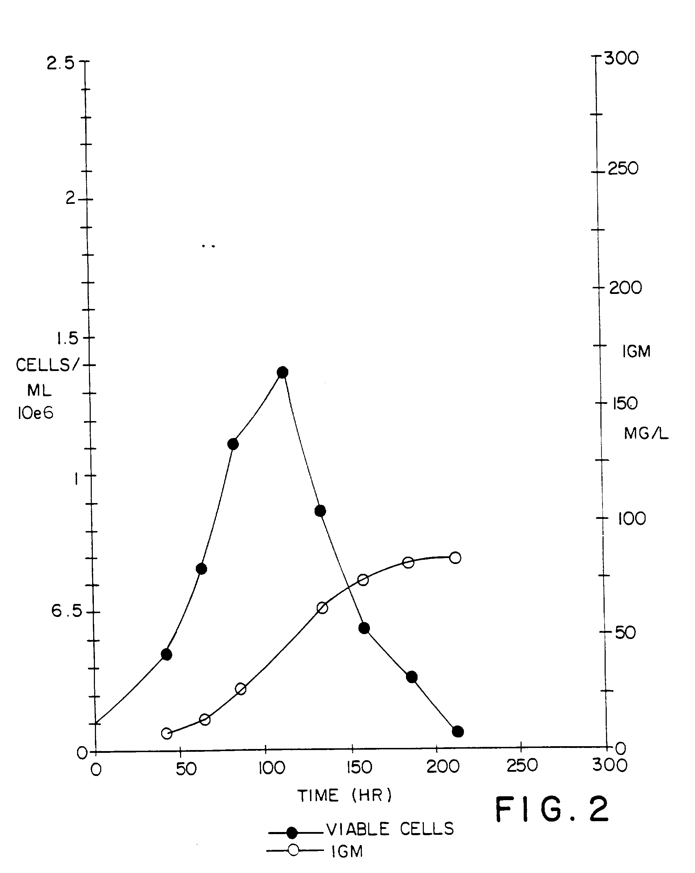

Figure 2 shows growth and product expression by the D234 hybridoma in the medium of Table I, column 1. Growth and product expression are similar to that in the commercially available HL-1 medium. The maximum viable cell density of 1.3 x 10⁶ cells/ml. The final antibody titre is 80 mg/L.

### Example 3

### Medium Containing Primary Supplement Addition Of Glutamine Before And During The Culture Period

Table I, column 4, shows a composition wherein the standard medium has been supplemented with the reagents of the Primary Supplement (glutamine at 8 mM, tryptophan at 140 mg/L, choline at 20 mg/L, ethanolamine at 10 mg/L and other amino acids increased in concentration by typically around 3 fold the concentration in the basal medium). In initial studies it was determined that the glucose level in the basal medium could be depleted and limit culture growth and longevity. Therefore, in addition to being included in the basal medium, additional glucose was added during the course of the culture period. Further, glutamine, in addition to being incorporated in the Primary Supplement to the medium at the start of culture, was also added at day 4, 8 and 12 such that the total glutamine supplied was equivalent to 20 mMoles per litre of culture.

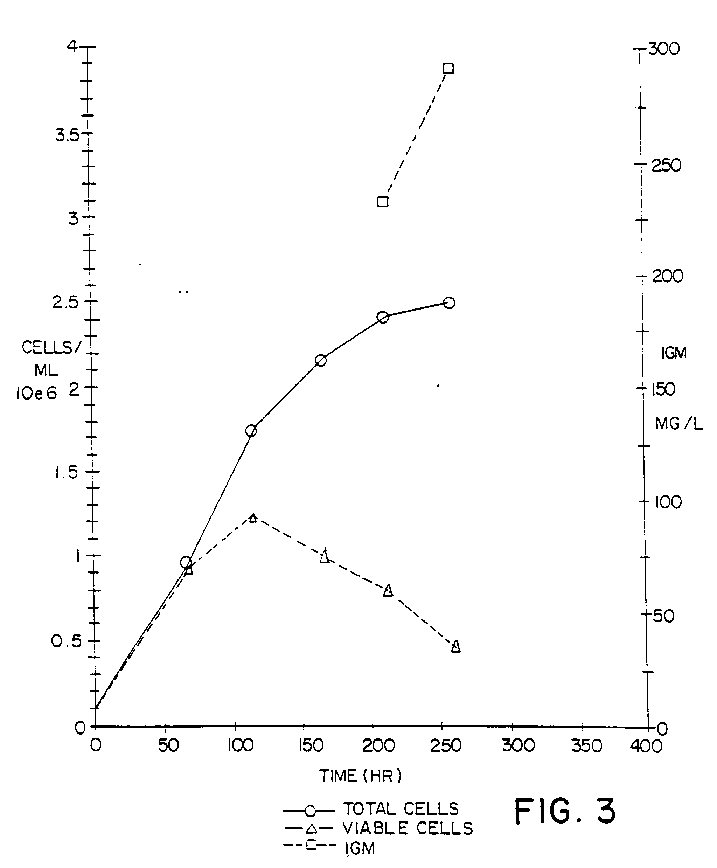

Figure 3 shows growth and product expression by D234 in the supplemented medium. Maximum viable cell density of 1.2 x 10⁶ cell/ml is reached at 120 hours, thereafter the culture is maintained in a pseudo-stationary phase, where cell growth approximately balances cell death such that the viable cell concentration declines only slowly over a period for 100 hours. The total cell density continues to rise to 2.5 x 10⁶ cells/ml. Cells continue to express product over the pseudo-stationary phase, and a final titre of 290 mg human antibody per litre is reached.

### Example 4

### Medium Containing Primary Supplement Addition Of Glutamine At The Start Of The Culture Period Only

D234 was grown in medium and conditions similar to those in Example 3, except that glutamine was included in the Primary Supplement to make the concentration of glutamine 20 mM at the start of culture, and glutamine was not added thereafter. Growth and product expression were equivalent to those in Example 3.

### Example 5

### Medium Containing Primary Supplement and Class I Reagent

Table I, column 2, shows a composition wherein standard medium has been supplemented with the reagents of the Primary Supplement as well as with monothioglycerol (a Class I reagent) at 10 mg/L. Glucose and glutamine were supplied as in Example 3.

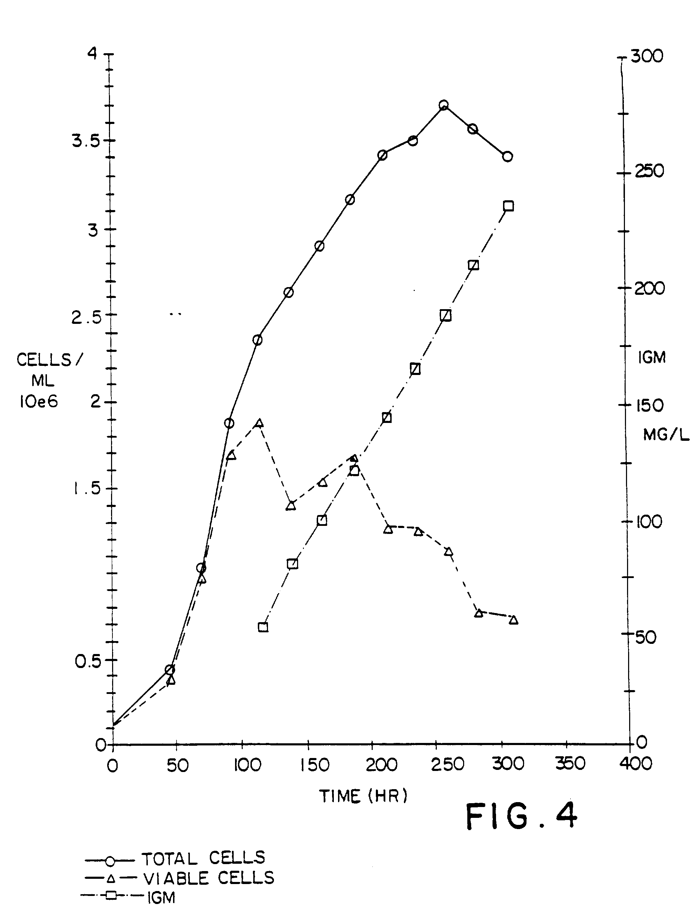

Figure 4 shows growth and product expression by D234 in the supplemented medium. Maximum viable cell density of 1.9 x 10⁶ cell/ml is reached at 120 hours. Thereafter, the culture is maintained in a pseudo-stationary phase, where cell growth rate approximately balances the rate of cell death such that the viable cell concentration declines only slowly over a period of 150 additional hours. Total cell density continues to climb to 3.8 x 10⁶ cells/ml. Cells continue to express product over the pseudo-stationary phase period and a final titre of 230 mg human antibody per L is reached.

### Example 6

### Supplement Used In Combination With Lipid Emulsion

Table I, column 3, shows a composition in which the typical cell culture medium has been supplemented with the reagents of the Primary Supplement (8 mM glutamine, 28 mg/L tryptophan, 22.8 mg/L choline, 0.8 mg/L ethanolamine, and other amino acids supplemented to typically around 3 fold the basal medium concentration), and with Class I reagents (1 mg/L monothioglycerol, vitamin B-12 and trace elements including molybdenum, cobalt, copper, zinc and iron). Additionally, the medium contains lipids (linoleic acid, tween 80, lecithin, cholesterol and vitamin E). Additionally, glucose and glutamine was added during the culture period as described in Example 3.

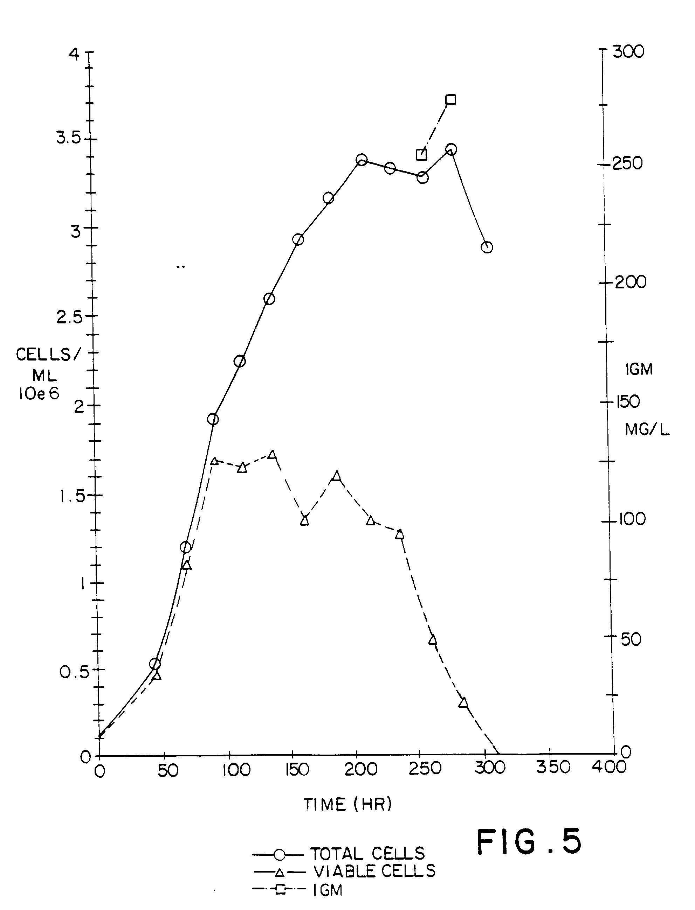

Figure 5 shows the growth and product expression by D234 in the supplemented medium. Maximum viable cell density of 1.7 x 10⁶ cells/ml is reached at 80 hours. Thereafter, the culture is maintained in a pseudo-stationary phase during which growth continues at approximately 50% of the maximum exponential phase growth rate which approximately balances the rate of death such that the viable cell concentration declines only slowly over a period of 150 additional hours. Total cell density continues to climb to 3.5 x 10⁶ cells/ml. Cells continue to express product over the pseudo-stationary phase period and a final titre of 275 mg human antibody per L is reached.

### Example 7

### Use Of Supplement In Fed-Batch Culture

The supplement of the present invention can be used beneficially in fed-batch, as well as in simple batch culture. Table I, column 5, shows the standard medium supplemented with reagents of the Primary Supplement and Class I reagents, as well as With lipids. This medium is similar to that of Example 6 (Table I, column 3).

This medium was used for growth of D234 in a fed-batch process. After 120 hours, additional supplement was added as shown in Table II. After 220 hours, glutamine was further supplemented with another 5.mmoles/L of culture.

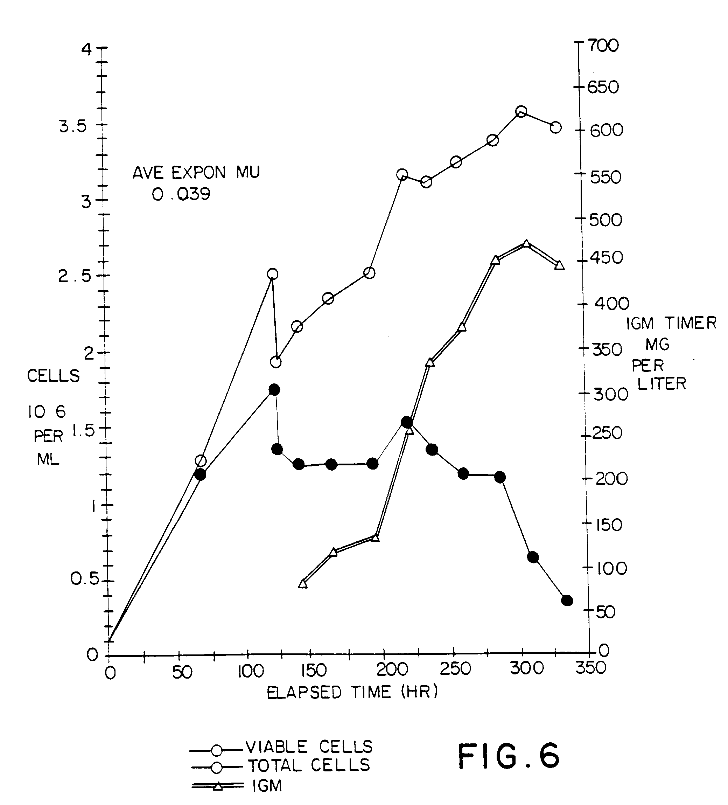

Figure 6 shows the growth and product expression by D234 in the supplemented fed-batch culture. Maximum viable cell density of 1.8 x 10⁶ cells/ml is reached at 130 hours. Thereafter, the culture is maintained in a pseudo-stationary phase during which growth continues at approximately 20% of the maximum exponential phase growth rate which approximately balances the rate of death such that the viable cell concentration declines only slowly over a period of 190 additional hours. Total cell density continues to climb to 3.7 x 10⁶ cells/ml. Cells continue to express product over the pseudo-stationary phase period and a final titre of 470 mg human antibody per L is reached.

Other feeding strategies have also been tested wherein the nutrients are added to a similar total final concentration as in the above sample, but are added at different schedules. Subdivision of the supplement addition into nearly equal daily additions beginning after 190 hours gives results similar to those described above. Slow continuous feeding also gives similar results.

Having described what applicants believe their invention to be, it will be appreciated that the invention should not be construed as limited in any manner whatsoever other than by the scope by the appended claims.

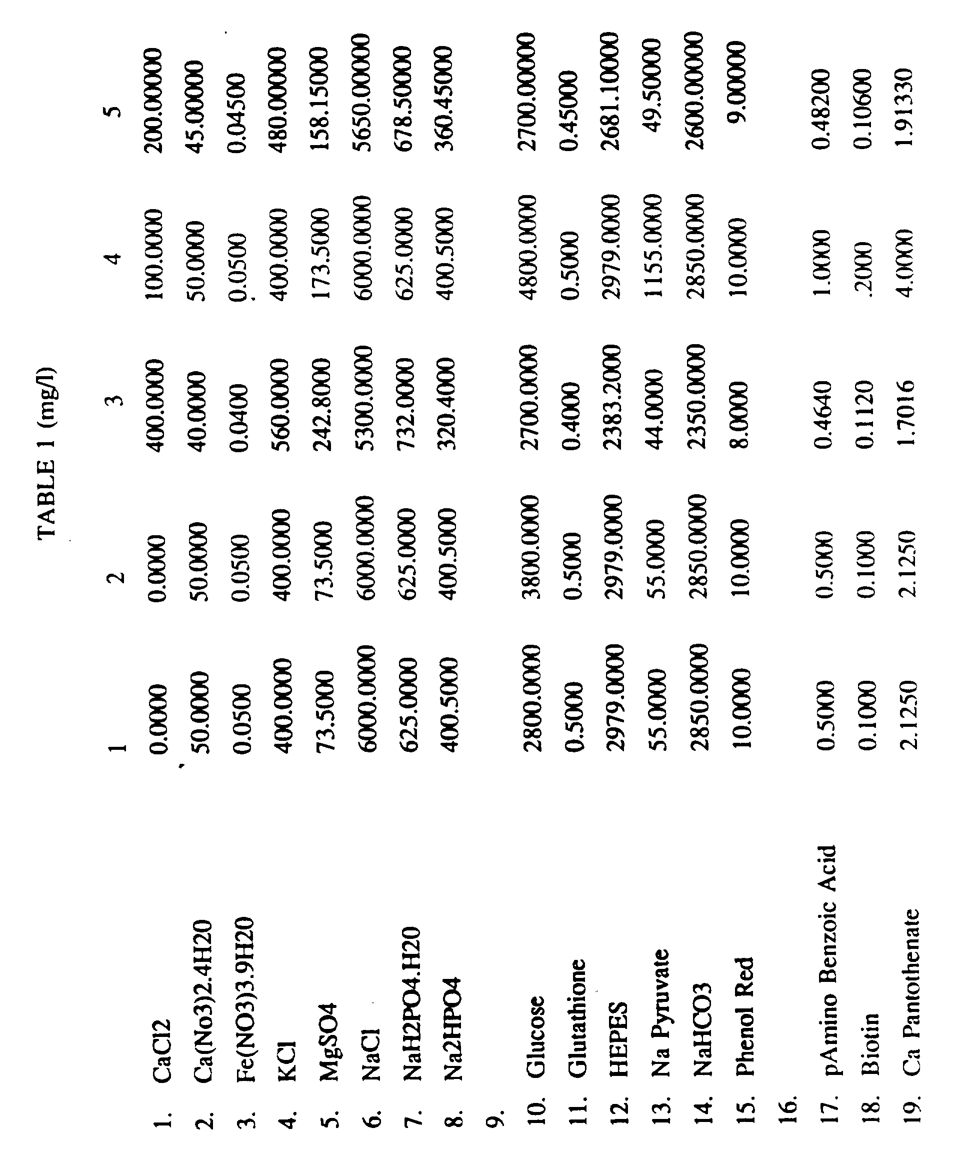

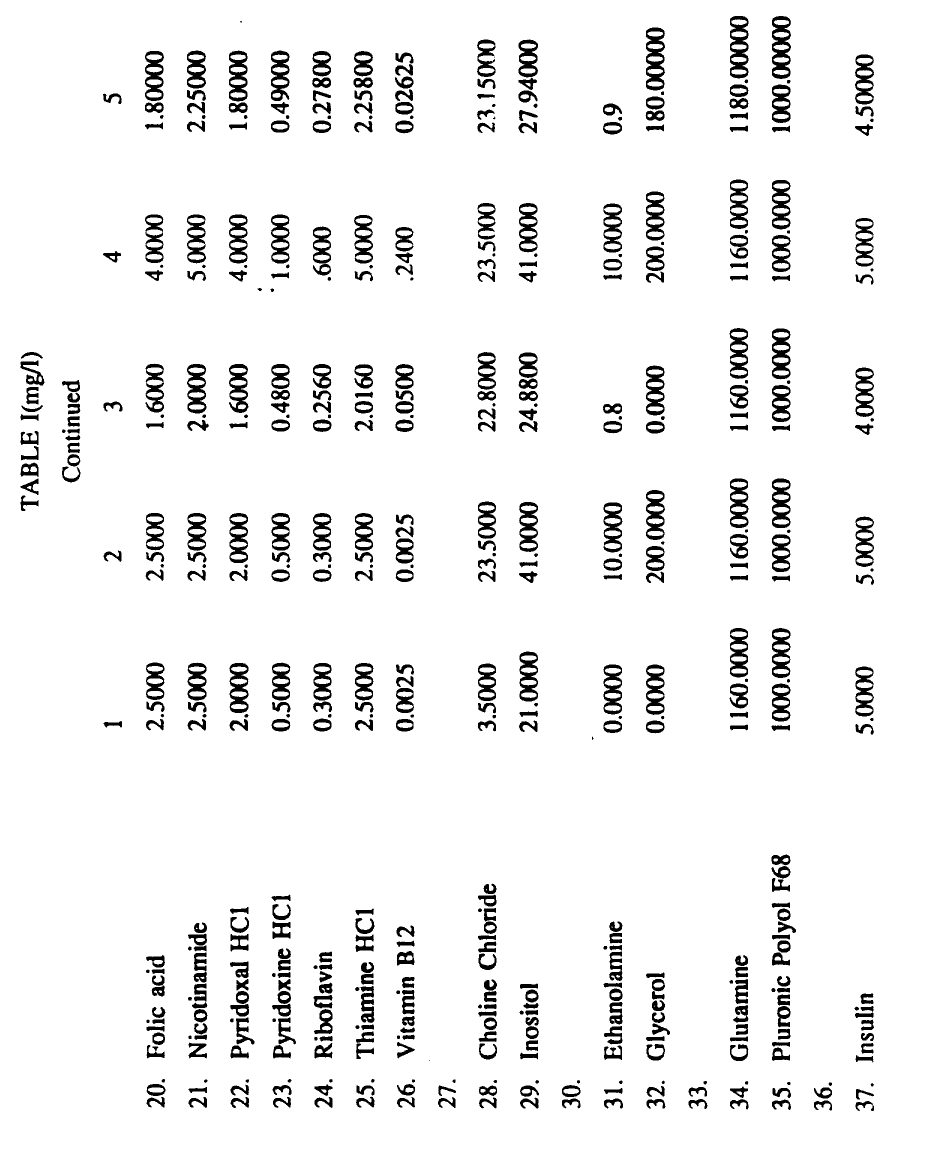

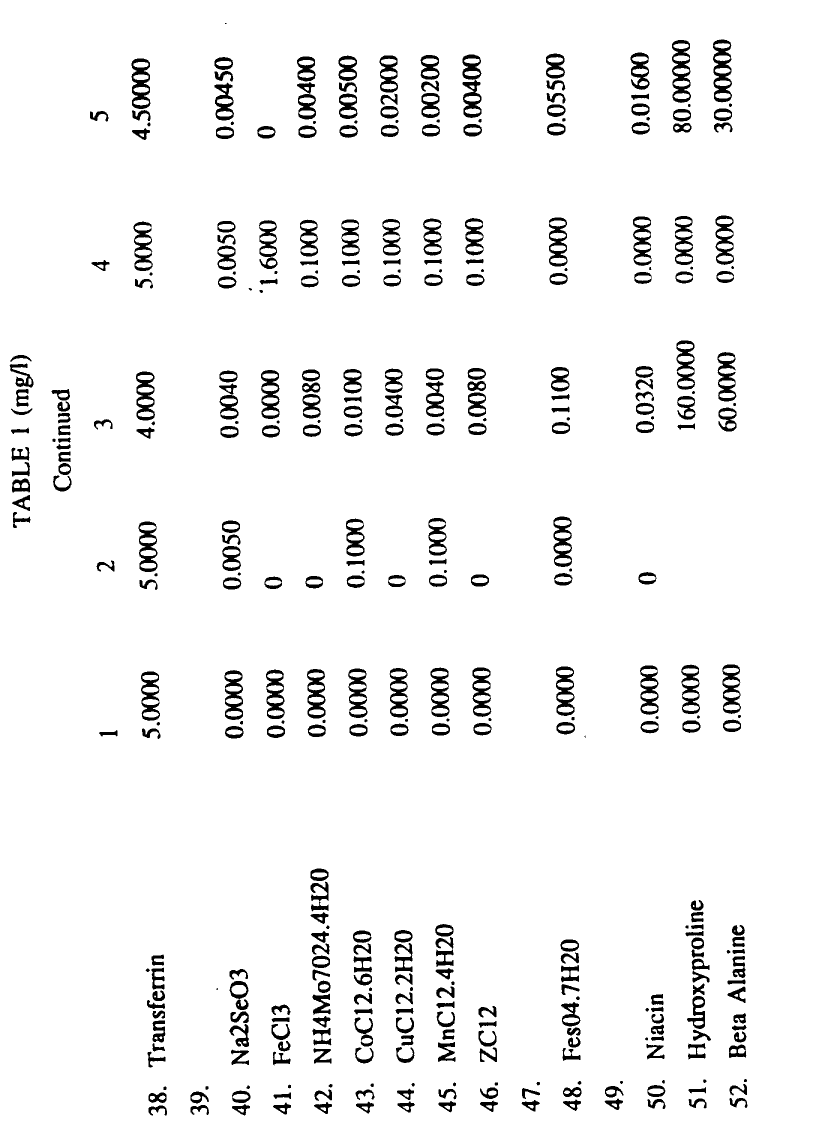

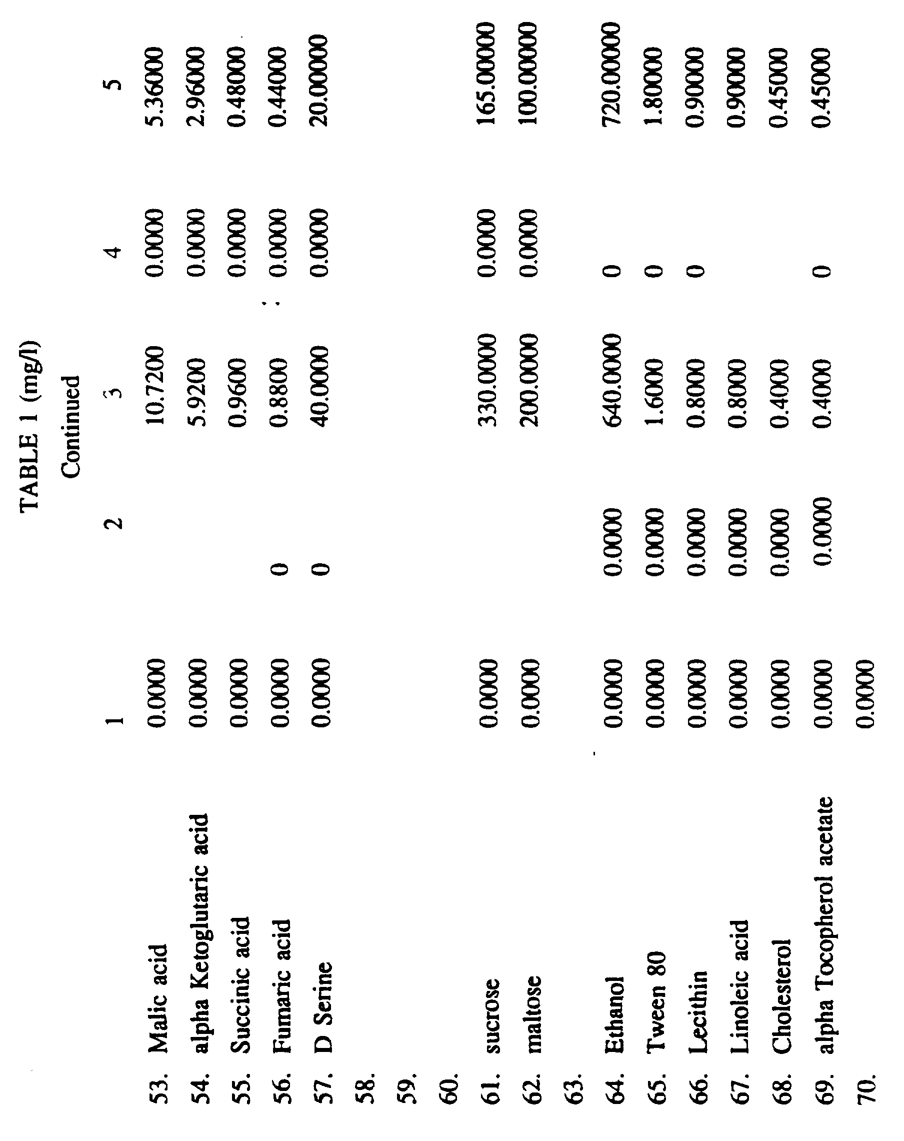

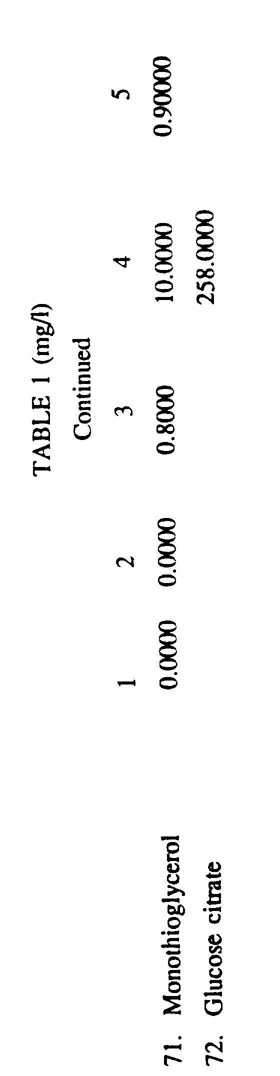

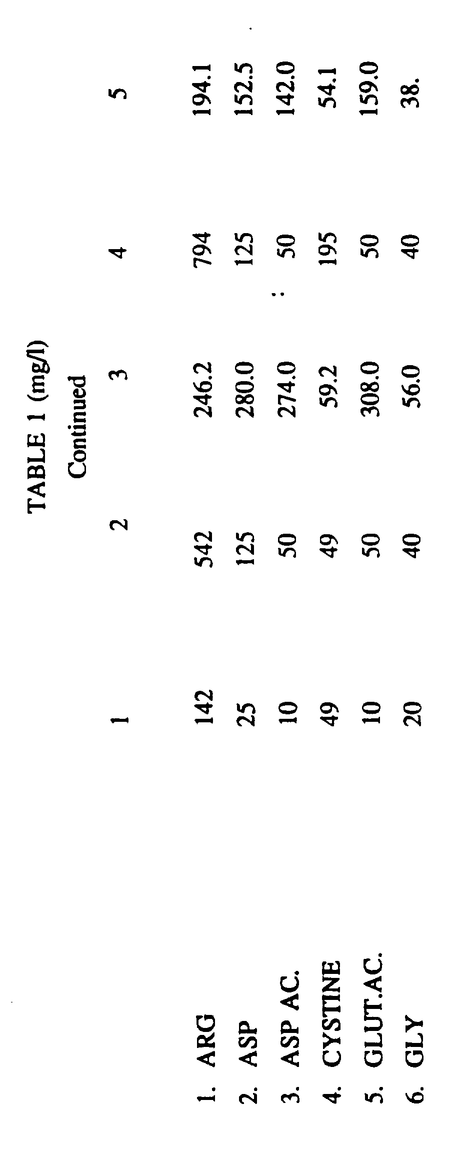

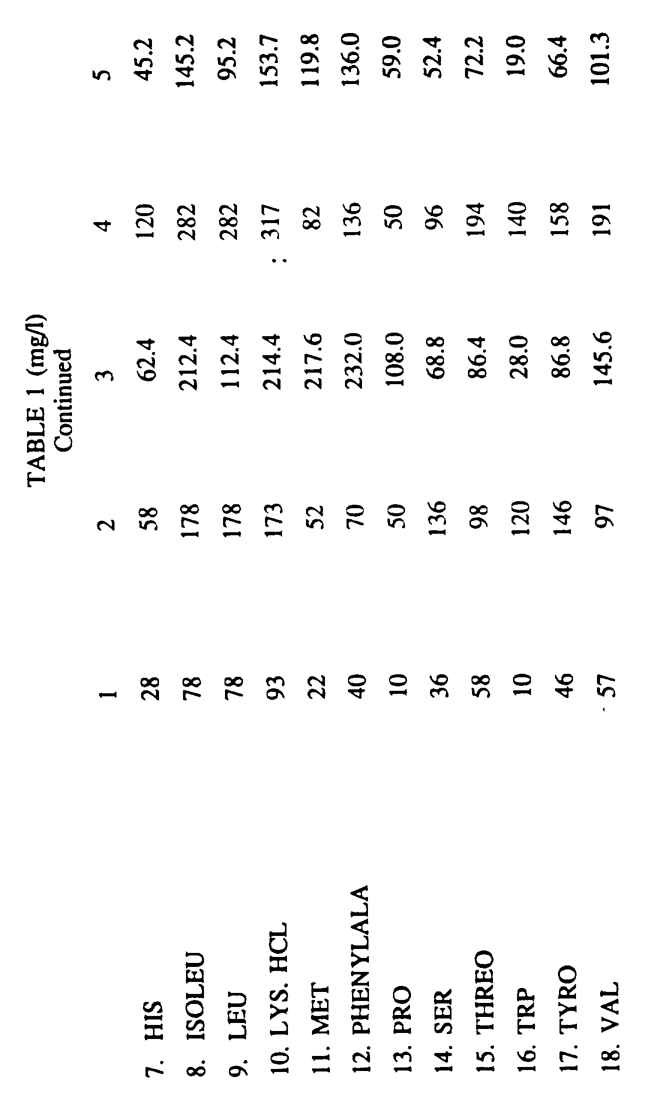

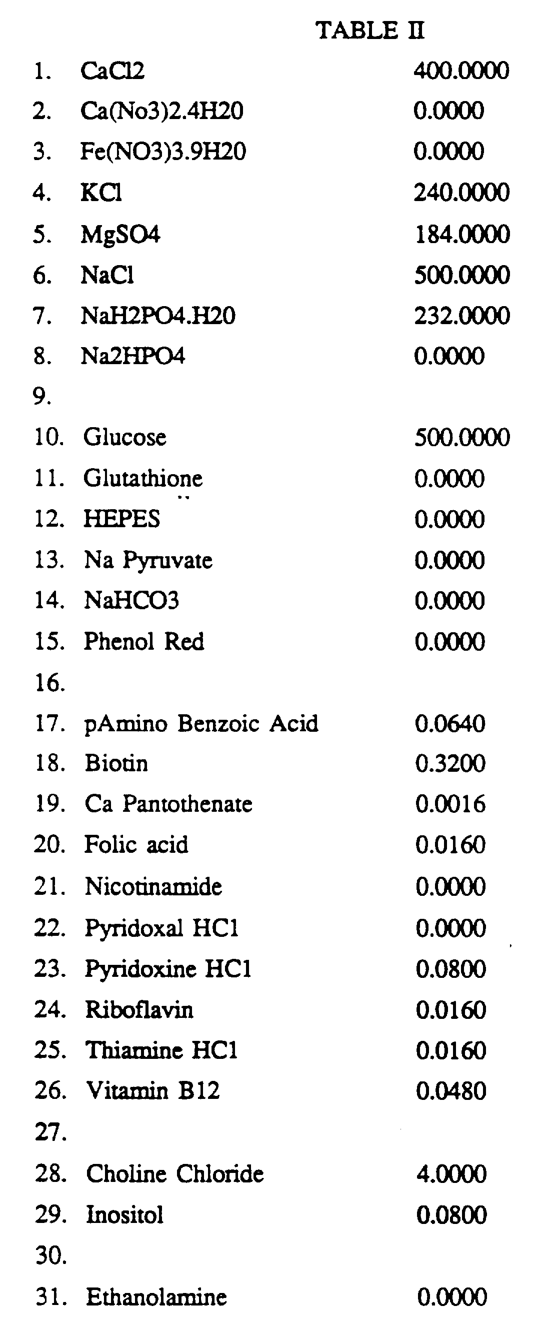

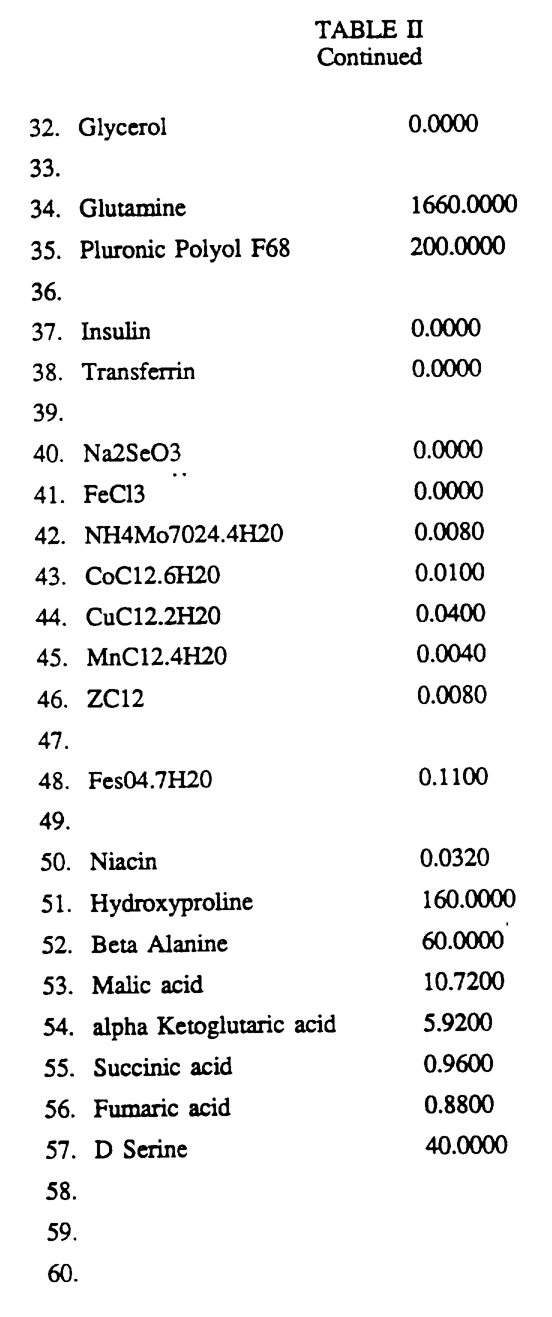

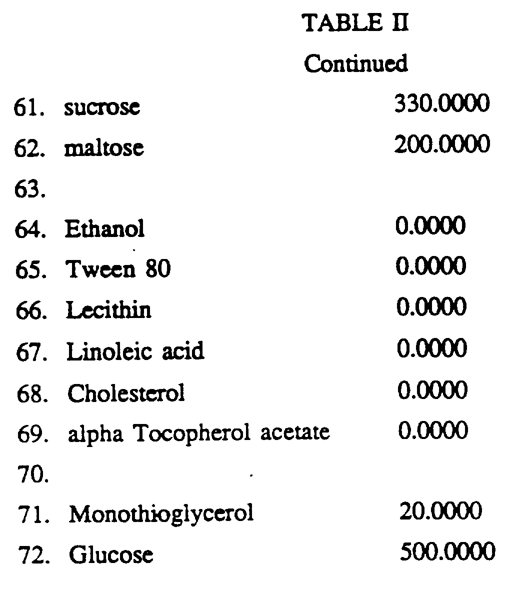

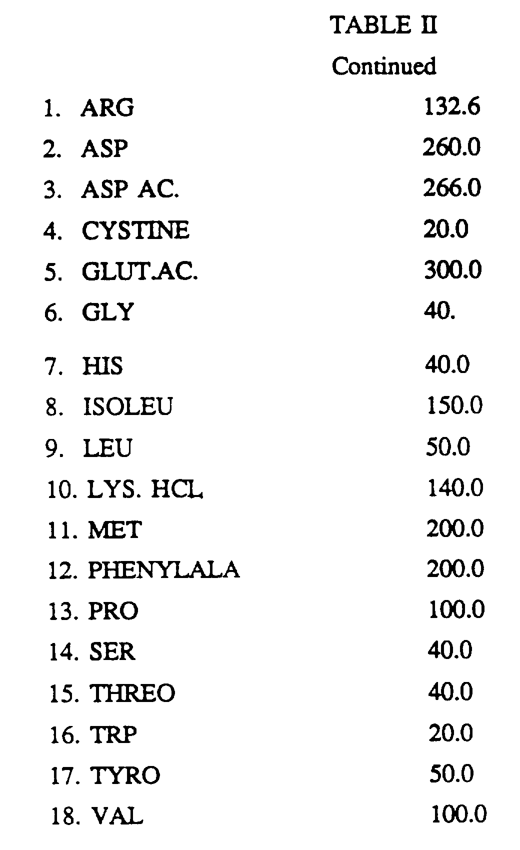

## Claims

1. A composition comprising
a Primary Supplement comprising:
(a) a first reagent selected from glutamine and glutamate;
(b) phospholipid precursors including at least choline and ethanolamine, the phospholipid precursors optionally being supplied in complex form; and
(c) amino acids;
and comprising the following Class I reagents: (i) a reducing agent, (ii) metal ions, (iii) a metal chelator, and (iv) vitamins,
wherein the Primary Supplement components and Class I reagents are supplied in amounts that effectively maintain cells in culture for a prolonged time in a pseudo-stationary phase growth phase.

2. A composition of claim 1, wherein the phospholipid precursors further include serine, inositol, and/or glycerol, and/or wherein the phospholipid precursors are supplied in complex form and include phosphoethanolamine. phosphocholine, phosphatidylcholine, and/or phosphatidylethanolamine.

3. A composition of claim 1 or claim 2, wherein:
(a) the amino acids comprise tryptophan at a concentration that when added to culture media is more than about 20 mg/L, but less than about 200 mg/L; and/or
(b) the glutamine or glutamate is present at a concentration that when added to culture media is above about 5 mM, but below about 40 mM; and/or
(c) the choline is present at a concentration that when added to culture media is greater than about 4 mg/L; and/or
(d) the ethanolamine is present at a concentration greater than about 1 mg/L, but less than about 100 mg/L.

4. A composition of any of claims 1 to 3, wherein reducing agent is included at a concentration greater than about 0.1 mg/L and less than about 100 mg/L and/or is selected from beta-mercaptoethanol. monothioglycerol and dithiothreitol.

5. A composition of any of claims 1 to 4, which further comprises one or more of the following features:
(a) the metal ions are selected from iron, calcium, manganese, molybdenum, cobalt, copper, zinc, and selenium,
(b) the metal ions are selected from iron, calcium, manganese, molybdenum, cobalt, copper, zinc, and selenium and the chelator is selected from transferrin, ferritin, albumin, pyridoxyl isonicetinoyl hydrazone, choline citrate and citrate, any citrate preferably being included in medium at a concentration of about 1-10 mM,
(c) the vitamins are selected from p-amino-benzoic acid, biotin, folic acid, folinic acid, nicotinamide, pantothenate, pyridoxine, riboflavin, flavin adenine dinucleotide, ascorbic acid, thiamine and vitamin B-12.

6. A composition of any of claims 1 to 5 and further comprising growth hormones, growth factors or serum.

7. A composition of claim 6 also comprising a lipid emulsion.

8. A composition of claim 7, wherein the lipid emulsion comprises a fatty acid, a nonionic detergent, a phospholipid, an antioxidant and cholesterol.

9. A medium for growing cells comprising a basal medium, a Primary Supplement comprising
(a) a first reagent selected from the group consisting of glutamine and glutamate:
(b) phospholipid precursors including at least ethanolamine and choline, the phospholipid precursors optionally being supplied in complex form; and
(c) amino acids
and Class I Reagents comprising:
(a) a reducing agent,
(b) metal ions,
(c) a metal chelator, and
(d) vitamins;
and glucose, wherein the components of the Primary Supplement, Class I reagents, and glucose are in amounts that effectively maintain the cells in culture for prolonged times in a pseudo-stationary growth phase.

10. A medium of claim 9 wherein the phospholipid precursors are supplied in complex form selected from phosphoethanolamine, phosphocholine, and phosphatidylethanolamine.

11. A medium of claim 9 or claim 10, wherein the reducing agent is monothioglycerol.

12. A medium of any of claims 9 to 11 which further comprises a Pluronic® polyol.

13. A medium of any of claims 9 to 12, wherein the basal medium is free of serum and proteins.

14. A medium of any of claims 9 to 13, wherein the metal ions comprise calcium. cobalt, copper, iron, manganese, magnesium, molybdenum, selenium and zinc.

15. A medium of any of claims 9 to 14 wherein the metal chelator is citrate.

16. A medium of any of claims 9 to 15, where the vitamins comprise nicotinamide and pyridoxine, and optionally biotin, pantothenate, folic acid, riboflavin, thiamine and vitamin B12.

17. A cell culture medium Primary Supplement comprising glutamine or glutamate, amino acids and phospholipid precursors, said phospholipid precursors optionally being supplied in complex form, in amounts that effectively maintain cells in culture for prolonged times in a pseudo-stationary growth phase, and wherein:
(a) the amino acids comprise tryptophan at a concentration that when added to culture media is more than about 20 mg/L, but less than about 200 mg/L;
(b) the glutamine or glutamate is present at a concentration that when added to culture media is above about 5 mM, but below about 40 mM;
(c) the choline is present at a concentration that when added to culture media is greater than about 4 mg/L; and
(d) the ethanolamine is present at a concentration greater than about 1 mg/L, but less than about 100 mg/L.

18. A Primary Supplement of claim 17 which further comprises the feature(s) of one or both of claims 2 and 16.

19. A cell culture medium supplement comprising a Primary Supplement of claim 17 or claim 18 and Class I reagents, the Class I reagents being present in effective amounts that co-act with the Primary Supplement to maintain cells in a pseudo-stationary phase, and comprising a reducing agent, trace metal ions and vitamins.

20. A supplement of claim 19 which further compnses the feature(s) of one or more of claims 4 to 8 or 10 to 15.

21. A method of growing cells, optionally antibody-secreting cells, comprising contacting said cells with a composition of any of claims 6 to 8.

22. A method of growing cells, comprising contacting the cells with cell culture medium wherein individually or in combination components of a composition of any one of claims 1 to 10, a medium of any one of claims 9 to 16, a Primary Supplement of claim 17 or claim 18 or a cell culture medium supplement of claim 19 or claim 20, are added to the cell culture medium over the time of cell culture to maintain the cells in a pseudo-stationary growth phase.

23. The use to grow cells and produce biochemicals therefrom of:
(i) composition of any one of claims 1 to 10;
(ii) a medium of any one of claims 9 to 16;
(iiii) a Primary Supplement of claim 17 or claim 18; or
(iv) a cell culture medium supplement of claim 19 or claim 20;
the components of the composition, medium or supplement, individually or in combination optionally being added to the cell culture medium over the time of cell culture to maintain the cells in a pseudo-stationary growth phase.

24. A method of producing a biochemical, comprising
(i) culturing cells in a composition of any of claims 6 to 8, or
(ii) adding to the culture medium the components of a composition, medium or supplement of claim 20(i), 20(ii), 20(iii) or 20(iv), the components, individually or in combination, optionally being added to the cell culture medium to maintain the cells in a pseudo-stationary growth phase.

## Patentansprüche

1. Zusammensetzung, umfassend
einen primären Zusatz, umfassend:
(a) ein erstes Reagenz, ausgewählt aus Glutamin und Glutamat;
(b) Phospholipidvorläufer, umfassend zumindest Cholin und Ethanolamin, wobei die Phospholipidvorläufer gegebenenfalls in Komplex-Form zugesetzt werden; und
(c) Aminosäuren;
und umfassend die folgenden Reagentien der Klasse I: (i) ein Reduktionsmittel, (ii) Metallionen, (iii) einen Metallchelator und (iv) Vitamine,
wobei die primären Zusatzkomponenten und die Klasse I-Reagentien in Mengen zugesetzt werden, die Zellen in Kultur für einen ausgedehnten Zeitraum in einer pseudostationären Wachstumsphase wirksam aufrechterhalten.

2. Zusammensetzung nach Anspruch 1, wobei die Phospholipidvorläufer weiterhin Serin, Inosit und/oder Glycerin umfassen, und/oder wobei die Phospholipidvorläufer in Komplex-Form zugesetzt werden und Phosphoethanolamin, Phosphocholin, Phosphatidylcholin und/oder Phosphatidylethanolamin umfassen.

3. Zusammensetzung nach Anspruch 1 oder 2, wobei:
(a) die Aminosäuren Tryptophan in einer Konzentration umfassen, die bei Zugabe zu Kulturmedien mehr als etwa 20 mg/l, aber weniger als etwa 200 mg/l beträgt; und/oder
(b) Glutamin oder Glutamat in einer Konzentration vorhanden ist, die bei Zugabe zu Kulturmedien oberhalb von etwa 5 mM, aber unterhalb von etwa 40 mM liegt; und/oder
(c) Cholin in einer Konzentration vorhanden ist, die bei Zugabe zu Kulturmedien größer als etwa 4 mg/l ist; und/oder
(d) Ethanolamin in einer Konzentration vorhanden ist, die größer als etwa 1 mg/l, aber geringer als etwa 100 mg/l ist.

4. Zusammensetzung nach einem der Ansprüche 1 bis 3, wobei ein Reduktionsmittel in einer Konzentration eingeschlossen ist, die größer als etwa 0,1 mg/l und geringer als etwa 100 mg/l ist, und/oder ausgewählt ist aus β-Mercaptoethanol, Monothioglycerin und Dithiothreit.

5. Zusammensetzung nach einem der Ansprüche 1 bis 4, die weiterhin ein oder mehrere der folgenden Merkmale umfaßt:
(a) die Metallionen sind ausgewählt aus Eisen, Calcium, Mangan, Molybdän, Kobalt, Kupfer, Zink und Selen,
(b) die Metallionen sind ausgewählt aus Eisen, Calcium, Mangan, Molybdän, Kobalt, Kupfer, Zink und Selen, und der Chelator ist ausgewählt aus Transferrin, Ferritin, Albumin, Pyridoxylisonicetinoylhydrazon, Cholincitrat und Citrat, wobei jedes beliebige Citrat vorzugsweise im Medium in einer Konzentration von etwa 1 bis 10 mM eingeschlossen ist,
(c) die Vitamine sind ausgewählt aus p-Aminobenzoesäure, Biotin, Folsäure, Folinsäure, Nicotinamid, Panthothenat, Pyridoxin, Riboflavin, Flavinadenindinucleotid, Ascorbinsäure, Thiamin und Vitamin B12.

6. Zusammensetzung nach einem der Ansprüche 1 bis 5, und weiterhin umfassend Wachstumshormone, Wachstumsfaktoren oder Serum.

7. Zusammensetzung nach Anspruch 6, auch eine Lipidemulsion umfassend.

8. Zusammensetzung nach Anspruch 7, wobei die Lipidemulsion eine Fettsäure, ein nicht-ionisches Detergens, ein Phospholipid, ein Antioxidans und Cholesterin umfaßt.

9. Medium zum Anziehen von Zellen, umfassend ein Basalmedium, einen primären Zusatz, umfassend
(a) ein erstes Reagens, ausgewählt aus Glutamin und Glutamat;
(b) Phospholipidvorläufer, umfassend zumindest Ethanolamin und Cholin, wobei die Phospholipidvorläufer gegebenenfalls in Komplex-Form zugesetzt werden; und
(c) Aminosäuren
und Klasse I-Reagentien, umfassend:
(a) ein Reduktionsmittel,
(b) Metallionen,
(c) einen Metallchelator und
(d) Vitamine;
und Glucose, wobei die Komponenten des primären Zusatzes, der Klasse I-Reagentien und Glucose in Beträgen vorhanden sind, die die Zellen in Kultur für ausgedehnte Zeiträume in einer pseudostationären Wachstumsphase wirksam aufrechterhalten.

10. Medium nach Anspruch 9, wobei die Phospholipidvorläufer in Komplex-Form zugesetzt werden, ausgewählt aus Phosphoethanolamin, Phosphocholin und Phosphatidylethanolamin.

11. Medium nach Anspruch 9 oder Anspruch 10, wobei das Reduktionsmittel Monothioglycerin ist.

12. Medium nach einem der Ansprüche 9 bis 11, das weiterhin ein Pluronic^{®}-Polyol umfaßt.

13. Medium nach einem der Ansprüche 9 bis 12, wobei das Basalmedium frei von Serum und Proteinen ist.

14. Medium nach einem der Ansprüche 9 bis 13, wobei die Metallionen Calcium, Kobalt, Kupfer, Eisen, Mangan, Magnesium, Molybdän, Selen und Zink umfassen.

15. Medium nach einem der Ansprüche 9 bis 14, wobei der Metallchelator Citrat ist.

16. Medium nach einem der Ansprüche 9 bis 15, wobei die Vitamine Nicotinamid und Pyridoxin umfassen und gegebenenfalls Biotin, Panthothenat, Folsäure, Riboflavin, Thiamin und Vitamin B12.

17. Primärer Zusatz für ein Zellkulturmedium, umfassend Glutamin oder Glutamat, Aminosäuren und Phospholipidvorläufer, wobei die Phospholipidvorläufer gegebenenfalls in Komplex-Form in Mengen zugesetzt werden, die Zellen in Kultur für ausgedehnte Zeiträume in einer pseudostationären Wachstumsphase wirksam aufrechterhalten, und wobei:
(a) die Aminosäuren Tryptophan in einer Konzentration umfassen, die bei Zugabe zu Kulturmedien mehr als etwa 20 mg/l, aber weniger als etwa 200 mg/l beträgt;
(b) Glutamin oder Glutamat in einer Konzentration vorhanden ist, die bei Zugabe zu Kulturmedien oberhalb von etwa 5 mM, aber unterhalb von etwa 40 mM liegt;
(c) Cholin in einer Konzentration vorhanden ist, die bei Zugabe zu Kulturmedien größer als etwa 4 mg/l ist; und
(d) Ethanolamin in einer Konzentration vorhanden ist, die größer als etwa 1 mg/l, aber weniger als etwa 100 mg/l ist.

18. Primärer Zusatz nach Anspruch 17, der weiterhin das (die) Merkmal(e) nach einem oder beiden Ansprüche 2 und 16 umfaßt.

19. Zusatz zu einem Zellkulturmedium, umfassend einen primären Zusatz nach Anspruch 17 oder Anspruch 18 und Klasse I-Reagentien, wobei die Klasse I-Reagentien in wirksamen Mengen vorhanden sind, die gemeinsam mit dem primären Zusatz wirken, zum Halten von Zellen in einer pseudostationären Phase, und umfassend ein Reduktionsmittel, Spurenmetallionen und Vitamine.

20. Zusatz nach Anspruch 19, der weiterhin das (die) Merkmal(e) nach einem oder mehreren der Ansprüche 4 bis 8 oder 10 bis 15 umfaßt.

21. Verfahren zum Anziehen von Zellen, gegebenenfalls von Antikörper-sezernierenden Zellen, umfassend das Inkontaktbringen der Zellen mit einer Zusammensetzung nach einem der Ansprüche 6 bis 8.

22. Verfahren zum Anziehen von Zellen, umfassend das Inkontaktbringen der Zellen mit Zellkulturmedium, wobei Komponenten einer Zusammensetzung nach einem der Ansprüche 1 bis 10, ein Medium nach einem der Ansprüche 9 bis 16, ein primärer Zusatz nach Anspruch 17 oder Anspruch 18 oder ein Zusatz zu einem Zellkulturmedium nach Anspruch 19 oder Anspruch 20 einzeln oder in Kombination zu dem Zellkulturmedium über den Zeitraum der Zellkultur zur Aufrechterhaltung der Zellen in einer pseudostationären Wachstumsphase zugefügt werden.

23. Verwendung zum Anziehen von Zellen und zur Herstellung von Biochemikalien davon von:
(i) einer Zusammensetzung nach einem der Ansprüche 1 bis 10;
(ii) einem Medium nach einem der Ansprüche 9 bis 16;
(iii) einem primären Zusatz nach Anspruch 17 oder Anspruch 18; oder
(iv) einem Zusatz zu einem Zellkulturmedium nach Anspruch 19 oder Anspruch 20;
wobei die Komponenten der Zusammensetzung, des Mediums oder des Zusatzes gegebenenfalls einzeln oder in Kombination zu dem Zellkulturmedium über den Zeitraum der Zellkultur zur Aufrechterhaltung der Zellen in einer pseudostationären Wachstumsphase hinzugefügt werden.

24. Verfahren zur Herstellung einer Biochemikalie, umfassend
(i) die Züchtung von Zellen in einer Zusammensetzung nach einem der Ansprüche 6 bis 8, oder
(ii) das Hinzufügen von Komponenten einer Zusammensetzung, eines Mediums oder eines Zusatzes nach Anspruch 20(i), 20(ii), 20(iii) oder 20(iv) zu dem Kulturmedium, wobei die Komponenten gegebenenfalls einzeln oder in Kombination zu dem Zellkulturmedium zur Aufrechterhaltung der Zellen in einer pseudostationären Wachstumsphase hinzugefügt werden.

## Revendications

1. Composition comprenant
un Additif Primordial comprenant:
(a) un premier réactif choisi entre la glutamine et le glutamate;
(b) des précurseurs des phospholipides incluant au moins la choline et l'éthanolamine, lesquels précurseurs des phospholipides sont éventuellement fournis sous forme de complexes; et
(c) des acides aminés;
et les réactifs de classe I suivants: (i) un agent réducteur, (ii) des ions métalliques, (iii) un chélateur de métaux, et (iv) des vitamines,
dans laquelle les composants de l'Additif Primordial et les réactifs de classe I sont fournis en quantités efficaces pour maintenir les cellules en culture en phase de croissance pseudo-stationnaire pendant une période prolongée.

2. Composition selon la revendication 1, dans laquelle les précurseurs des phospholipides incluent en outre la sérine, l'inositol et/ou le glycérol et/ou où les précurseurs des phospholipides sont fournis sous forme de complexes et incluent la phosphoéthanolamine, la phosphocholine, la phosphatidylcholine et/ou la phosphatidyléthanolamine.

3. Composition selon la revendication 1 ou 2, dans laquelle:
(a) les acides aminés comprennent le tryptophane à une concentration finale dans le milieu de culture supérieure à environ 20 mg/l, mais inférieure à environ 200 mg/l; et/ ou
(b) la glutamine ou le glutamate est présent à une concentration finale dans le milieu de culture supérieure à environ 5 mM, mais inférieure à environ 40 mM; et/ou
(c) la choline est présente à une concentration finale dans le milieu de culture supérieure à environ 4 mg/l; et/ou
(d) l'éthanolamine est présente à une concentration supérieure à environ 1 mg/l, mais inférieure à environ 100 mg/l;

4. Composition selon l'une des revendications 1 à 3, dans laquelle l'agent réducteur est inclus à une concentration supérieure à environ 0,1 mg/l, mais inférieure à 100 mg/l; et/ou est sélectionné parmi le bêta-mercaptoéthanol, le monothioglycérol et le dithiothréitol.

5. Composition selon l'une des revendications 1 à 4, qui répond, en outre, à une ou plusieurs des caractéristiques suivantes:
(a) les ions métalliques sont choisis entre le fer, le calcium, le manganèse, le molybdène, le cobalt, le cuivre, le zinc et le sélénium,
(b) les ions métalliques sont sélectionnés parmi le fer, le calcium, le manganèse, le molybdène, le cobalt, le cuivre, le zinc et le sélénium et le chélateur est choisi parmi la transferrine, la ferritine, l'albumine, le pyridoxyl isonicetinoyl hydrazone, le citrate de choline et un citrate, le citrate, quel qu'il soit, étant de préférence présent dans le milieu à une concentration de 1 à 10 mM environ,
(c) les vitamines sont choisies parmi l'acide-p-benzoïque la biotine, l'acide folique, l'acide folinique, le nicotinamide, le pantothénate, la pyridoxine, la riboflavine, le dinucléotide flavine-adénine, l'acide ascorbique, la thiamine et la vitamine B-12.

6. Composition selon l'une des revendications 1 à 5, comprenant de surcroît des hormones de croissance, des facteurs de croissance ou du sérum.

7. Composition selon la revendication 6 comprenant également une émulsion lipidique.

8. Composition selon la revendication 7, dans laquelle l'émulsion lipidique comprend un acide gras, un détergent non ionique, un phospholipide, un antioxydant et du cholestérol.

9. Milieu de culture de cellules comprenant un milieu de base, un Additif Primordial comprenant:
(a) un premier réactif choisi entre la glutamine et le glutamate;
(b) des précurseurs des phospholipides incluant au moins la choline et l'éthanolamine, lesquels sont éventuellement fournis sous forme de complexes; et
(c) des acides aminés;
et les réactifs de classe I suivants comprenant:
(a) un agent réducteur,
(b) des ions métalliques,
(c) un chélateur de métaux, et
(d) des vitamines;
et du glucose, dans lequel les composants de l'Additif Primordial, les réactifs de classe I et le glucose sont fournis en quantités efficaces pour maintenir les cellules en culture en phase de croissance pseudo-stationnaire pendant une période prolongée.

10. Milieu selon la revendication 9, dans lequel les précurseurs des phospholipides sont fournis sous une forme complexe et sélectionnés parmi la phosphoéthanolamine, la phosphocholine et la phosphatidyléthanolamine.

11. Milieu de la revendication 9 ou 10, dans lequel l'agent réducteur est le monothioglycérol.

12. Milieu selon l'une des revendications 9 à 11, qui comprend également du Pluronic ® polyol.

13. Milieu selon l'une des revendications 9 à 12, dans lequel le milieu de base ne contient ni sérum ni protéine.

14. Milieu selon l'une des revendications 9 à 13, dans lequel les ions métalliques comprennent le calcium, le cobalt, le cuivre, le fer, le manganèse, le magnésium, le molybdène, le sélénium et le zinc.

15. Milieu selon l'une des revendications 9 à 14, dans lequel le chélateur est un citrate.

16. Milieu selon l'une des revendications 9 à 15, dans lequel les vitamines comprennent la nicotinamide et la pyridoxine et éventuellement la biotine, le panthothénate, l'acide folique, la riboflavine, la thiamine et la vitamine B12.

17. Additif Primordial pour milieu de culture de cellules comprenant de la glutamine ou du glutamate, des acides aminés et des précurseurs des phospholipides, lesdits précurseurs de phospholipides étant éventuellement fournis sous forme d'un complexe en quantité efficace pour maintenir les cellules en culture en phase de croissance pseudo-stationnaire pendant une période prolongée et dans lequel:
(a) les acides aminés comprennent le tryptophane à une concentration finale dans le milieu de culture supérieure à environ 20 mg/l, mais inférieure à environ 200 mg/l; et/ou
(b) la glutamine ou le glutamate est présent à une concentration finale dans le milieu de culture supérieure à environ 5 mM, mais inférieure à environ 40 mM; et/ou
(c) la choline est présente à une concentration finale dans le milieu de culture supérieure à environ 4 mg/l; et/ou
(d) l'éthanolamine est présente à une concentration supérieure à environ 1 mg/l, mais inférieure à environ 100 mg/l;

18. Additif Primordial selon la revendication 17, qui répond en outre à la ou aux caractéristiques de l'une des revendications 2 et 16 ou des deux.

19. Milieu de culture de cellules comprenant un Additif Primordial selon la revendication 17 ou la revendication 18 et des réactifs de Classe I, ces réactifs de classe I étant présents en quantités efficaces pour maintenir, en agissant en association avec l'Additif Primordial, les cellules en culture en phase de croissance pseudo-stationnaire pendant une période prolongée et comprenant un agent réducteur, de très faibles quantités d'ions métalliques et des vitamines.

20. Additif selon la revendication 19, qui répond en outre à une ou à plusieurs des caractéristiques de l'une ou de plusieurs des revendications 4 à 8 ou 10 à 15.

21. Procédé de culture de cellules, éventuellement de cellules sécrétrices d'anticorps, comprenant la mise en contact desdites cellules avec une composition de l'une des revendications 6 à 8.

22. Procédé de culture de cellules comprenant la mise en contact desdites cellules avec un milieu de culture dans lequel, individuellement ou en combinaison, les composants d'une composition selon l'une des revendications 1 à 10, un milieu selon l'une des revendications 9 à 16, un Additif Primordial selon la revendication 17 ou la revendication 18 ou un additif de milieu de culture selon la revendication 19 ou la revendication 20 sont ajoutés à un milieu de culture de cellules pendant la durée de la culture pour maintenir les cellules en phase de croissance pseudo-stationnaire.

23. Utilisation pour cultiver des cellules et produire des produits biochimiques à partir de ces cellules de:
(i) une composition selon l'une des revendications 1 à 10;
(ii) un milieu selon l'une des revendications 9 à 16;
(iii) un Additif Primordial selon la revendication 17 ou la revendication 18 ou
(iv) un additif au milieu de culture selon la revendication 19 ou la revendication 20;
les composants de la composition, du milieu ou de l'additif étant éventuellement ajoutés au milieu de culture de cellules, individuellement ou en combinaison, pendant la durée de la culture pour maintenir les cellules en phase de croissance pseudo-stationnaire.

24. Procédé de production d'un produit biochimique comprenant
(i) la culture des cellules dans une composition selon l'une des revendications 6 à 8 ou
(ii) l'addition au milieu de culture des composants d'une composition, d'un milieu ou d'un additif selon la revendication 20(i), 20(ii), 20(iii) ou (20iv), les composants étant éventuellement ajoutés au milieu de culture de cellules, individuellement ou en combinaison, pour maintenir les cellules en phase de croissance pseudo-stationnaire.
